# EUROPEAN PATENT SPECIFICATION

(11) **EP 0 880 594 B1**
(45) Date of publication and mention of the grant of the patent: **23.10.2002**
(21) Application number: 97901179.8
(22) Date of filing: 23.01.1997
(51) Int. Cl.: C12N 15/86, C12N 15/87, A61K 48/00

(54) **RETROVIRAL VECTOR AND ITS USE IN GENE THERAPY**
RETROVIRALER VEKTOR UND DESSEN VERWENDUNG IN GENTHERAPIE
VECTEUR DE RETROVIRUS ET SON UTILISATION EN THERAPIE GENIQUE

(30) Priority: 23.01.1996 GB 9601336; 04.10.1996 GB 9620759
(43) Date of publication of application: 02.12.1998
(73) Proprietor: Oxford Biomedica (UK) Limited, Oxford 0X4 4GA (GB)
(72) Inventor: KINGSMAN, Alan John, Oxon OX5 2SF (GB); KINGSMAN, Susan Mary, Oxon OX5 2SF (GB)
(74) Representative: Harding, Charles Thomas
(86) International application number: GB9700210
(87) International publication number: WO97027310

(56) References cited:
- WO-A-94/29438
- WO-A-95/22617
- NUCLEIC ACIDS RESEARCH, vol. 23, no. 4, 25 February 1995, pages 628-633, XP000569533 SONEOKA Y ET AL: "A TRANSIENT THREE-PLASMID EXPRESSION SYSTEM FOR THE PRODUCTION OF HIGH TITER RETROVIRAL VECTORS" cited in the application
- HEMATOLOGY/ONCOLOGY CLINICS OF NORTH AMERICA, vol. 9, 1995, pages 91-113, XP000653407 LEE, J H AND KLEIN, H G : "CELLULAR GENE THERAPY"
- CURRENT OPINION IN GENETICS AND DEVELOPMENT, vol. 3, 1993, pages 110-114, XP000653497 GROSSMAN, M AND WILSON, J M: "RETROVIRUSES: DELIVERY VEHICLE TO THE LIVER"

## Description

This invention relates to the use of sets of DNA sequences encoding retroviral vector particles in formulations for gene therapy, and to producer cells, comprising such sets of DNA sequences.

### INTRODUCTION AND PRIOR ART

A number of diseases are amenable to treatment by the delivery of therapeutic nucleic acids to patient's cells. This is referred to as gene therapy (reviewed extensively in Lever and Goodfellow 1995; Culver 1995; Ledley 1995). To achieve gene therapy there must be a method of delivering genes to the patient's cells and additional methods to ensure the effective production of any therapeutic genes. There are two general approaches to achieve gene delivery; these are non-viral delivery and virus-mediated gene delivery. The best characterised virus-mediated gene delivery system uses replication defective retroviruses to stably introduce genes into patients cells. A major disadvantage of non-viral delivery is that the DNA is confined to the initial target cells and is short lived which, for chronic disease, necessitates repeated treatments with the DNA. A major disadvantage of retroviral vectors is that efficient gene transfer is only achieved by transducing cells *ex vivo* and introducing either the transduced cell population back into the patient or grafting in a cell line that is engineered to release retroviral vector particles. These procedures require significant surgical procedure and manipulation of cells. In addition transduction of patients cells with retroviral vector particles is inefficient.

The various known technologies involved in the field of the invention are described in more detail below.

### 1. The production of retroviral vectors from multiple separate DNA sequences

It is known that the separate expression of the components of a retroviral vector on separate DNA sequences cointroduced into the same cell will yield retroviral particles carrying defective retroviral genomes that carry therapeutic genes (e.g. Reviewed by Miller 1992). This cell is referred to as the producer cell. There are two common procedures for generating producer cells. In one, the sequences encoding retroviral Gag, Pol and Env proteins are introduced into the cell and stably integrated into the cell genome; a stable cell line is produced which is referred to as the packaging cell line. The retroviral vector genome is then introduced into the packaging cell line by transfection or transduction to create a stable cell line that has all of the DNA sequences required to produce a retroviral vector particle. The second approach is to introduce the three different DNA sequences that are required to produce a retroviral vector particle i.e. the *env* coding sequences, the *gag-pol* coding sequence and the defective retroviral genome into the cell at the same time by transient transfection and the procedure is referred to as transient triple transfection (Landau & Littman 1992; Pear et al 1993;). The triple transfection procedure procedure has been optimised (Soneoka et al 1995; Finer et al 1994). WO 94/29438 describes the production of producer cells *in vitro* using this multiple DNA transient transfection method and describes the use of these producer cells *in vitro* to transfer retroviral particles to human cells of any lineage that have been removed from a patient. WO 94/19478 describes the use of novel cell lines for producing high titre retroviral stocks following the transient transfection of one or more retroviral plasmids into a packaging cell line. This also describes only the transfer of retroviruses to target cells *in vitro.* The transfer of retroviruses from a producer cell to a target cell *in vitro* is referred to as cocultivation and it is a well established procedure for introducing retroviruses into cells *in vitro.*

### 2. DNA mediated gene delivery in vivo.

The delivery of genes into a variety of different cells in man or animals using naked DNA or DNA associated with a non-viral delivery system has been well described (reviewed by Ledley 1995). The simplest method involves injecting naked DNA into tissues where it is taken up by a proportion of cells and the genes contained in the DNA are expressed to produce proteins in these cells (Dubensky et al 1984; Wolffe et al 1990).

The DNA may be delivered by biolistics; in this procedure metal particles are coated with DNA and projected at high velocity into cells by a high pressure device (e.g. Yang et al 1990). The DNA may be coupled to chemical agents that optimise uptake into cells e.g polylysine or to components of viral particles e.g. adenovirus particles or penton protein or to ligands for specific cognate receptors. The DNA may be encapsulated in liposomes or complexed with cationic lipids (e.g.Hyde et al 1993). Irrespective of how the DNA is delivered by these non-viral methods the seminal feature is that there is no transfer of DNA from the originally transfected cells to other cells except possibly by transfer to daughter cells after cell division. Furthermore the introduced gene is not guaranteed to be permanently maintained in the target cells.

### 3. Retrovirus mediated gene delivery.

The use of defective retrovirus vectors to deliver genes to target cells is well documented (reviewed by Morgan and Anderson 1993). Defective retroviruses are used to transduce cells that have been removed from the body (*ex vivo* gene delivery) or they can be delivered to tissues *in situ* (*in vivo* gene delivery). The vectors introduce DNA into a cell and it is stably incorporated into the host cell genome where it is expressed to produce any therapeutic gene contained within it. There is no dissemination of the therapeutic gene because retroviral vector mediated gene transfer is a one step event that affects only the initial target cell. *In vivo* gene delivery is not widely used because gene delivery is inefficient largely because the retroviral particles delivered in this way are rapidly cleared from the site of treatment and there is no extended exposure of the cells to viral particles. For example when retroviral vector particles were injected into the brains of rats that were carrying glial tumours only a very few cells were transduced by the vectors due to the short, 2-4hrs, half life of the retroviral particles (Short et al 1990).

### 4. Implantation of producer cells in target tissues

It has been reported that a producer cell that has been created *in vitro* can be implanted into a tissue *in situ* (Short et al 1990). The producer cell releases retroviral vector particles which then transduce neighbouring cells. In this procedure a producer cell is created by the stable transformation of the cell with the DNA sequences specifiying retroviral components *in vitro*. The cell is cultured *in vitro* and then surgically implanted in the patient. The producer cell is foreign and may be short lived due to destruction by the immune system.

Clearly there is a need for improved ways and means for introducing therapeutic genes into patients. Gene therapy would be significantly simplified if stable introduction of DNA into patient cells could be achieved following non-viral DNA delivery and if the effectiveness of non-viral DNA delivery could be improved.

WO 96/17053 describes an adenoviral vector capable of tissue-specific replication due to a regulatory sequence operably linked to the coding region of a gene essential for vector replication. The vector can be used to distribute a polynucleotide in a tissue *in vivo*.

WO 95/22617 describes a retrovirus delivery system in which the vector genome contains a therapeutically active gene in place of the *env* gene, but is otherwise identical to the wild type genome. It is suggested that by introducing an *env* gene into the target cell, retrovirus vector particles may then be produced *in situ*.

### THE INVENTION

The first aspect of the invention relates to a method for introducing a therapeutically active gene into a target cell. In a first embodiment, the invention provides the use of set of DNA sequences comprising:
a first DNA sequence encoding a replication defective retroviral vector, which comprises
   (a) a defective retroviral genome lacking functional *env* and functional *gag-pol* genes but having the remaining components essential for retroviral function; and
   (b) a therapeutically active gene; and
a second DNA sequence capable of encoding packaging components env and gag-pol, wherein the DNA sequence encoding env is present on a separate construct to the DNA sequence encoding gag-pol
in the manufacture of a medicament for use in a method for introducing the therapeutically active gene into a target cell in a subject which comprises the following steps:
(i) introducing the set of DNA sequences into a cell isolated from the subject which is thereby converted into a producer cell, capable of producing a replication defective retroviral vector;
(ii) reimplanting said producer cell into the subject whereby replication defective retroviral vector particles are produced *in vivo*, and the therapeutically active gene is delivered to the target cell *in vivo*.

In a second embodiment, the invention provides the use of a set of DNA sequences comprising:
a first DNA sequence encoding a replication defective retroviral vector, which comprises
   (a) a defective retroviral genome lacking functional *env* and functional *gag-pol* genes but having the remaining components essential for retroviral function; and
   (b) a therapeutically active gene; and
a second DNA sequence capable of encoding packaging components env and gag-pol, wherein the DNA sequence encoding env is present on a separate construct to the DNA sequence encoding gag-pol
in the manufacture of a medicament for use in a method for introducing the therapeutically active gene into a target cell in a subject which comprises the following step:
introducing the set of DNA sequences to a subject's cell by *in vivo* delivery, whereby the subject's cell is converted into a producer cell *in vivo* which is capable of producing replication defective retroviral vector particles, whereby the therapeutically active gene is delivered to the target cell *in vivo*.

Preferably the producer cell is of the same type as the target cell.

In another aspect, the invention provides a producer cell capable of producing a replication defective retroviral vector in an infective retroviral vector particle, the producer cell comprising a set of DNA sequences, comprising:
a first DNA sequence encoding a replication defective retroviral vector, which comprises
   (i) a defective retroviral genome lacking functional *env* and functional *gag-pol* genes but having the remaining components essential for retroviral function, and
   (ii) at least one therapeutically active gene; and
a second DNA sequence encoding a DNA sequence capable of encoding packaging components env and gag-pol wherein the DNA sequence encoding env is present on a separate construct to the DNA sequence encoding gag-pol
which producer cell is a fresh cell isolated from a subject and, when reimplanted into the subject, is capable of delivering the therapeutically active gene to a target cell within the subject by *in vivo* production of replication defective retroviral vector particles.

The producer cell may be an immune cell.

As will be explained below in more detail, in the method of the first aspect of the invention conversion of the fresh mammalian cells to producer cells may be carried out either *in vivo* or *in vitro*. In the *in vitro* case, the producer cells will be suitable for implanting into a patient, and are preferably cells from the patient into whom it is intended to reimplant them.

It is particularly preferred that the producer cells which are made in the method of the first aspect of the invention are of a target cell type for which the therapeutically active gene is intended. This avoids the need to introduce any exogenous cells into the target area in a patient.

However, the producer cells may alternatively be cells capable of delivering the therapeutically active gene to the target cell. Cells of the immune system such as macrophages or tumour infiltrating lymphocytes could be converted to producer cells; this would need to be carried out *in vitro.* When reintroduced into the patent these mobile producer cells will infiltrate organs or tissues and retroviral vector particles from the producer cells will infect those organs or tissues.

The term "fresh mammalian cells" as used here refers to mammalian cells which are in their natural state, or as close as possible to their natural state. Cells which have been extensively cultured *in vitro*, including cell lines, are not considered to be fresh cells. Fresh cells as referred to herein which have been removed from an organism will usually be primary cells in the sense that they are in a primary culture of cells prepared directly from the tissues of an organism and have not been subcultured.

It is an important feature of this invention that the replication defective retroviral vector does not encode any of the structural components of retroviral vector particles. The vector thus allows for the insertion of the therapeutically active gene or genes into the target cell genome, without structural genes encoding Env and Gag-Pol. This avoids problems associated with expression of viral components in the target cells, such as undesired immune responses to those components. For example, cytotoxic T cell responses directed against the products of other foreign genes will be avoided.

As will be evident to those skilled in the art; safety considerations to avoid generation of replication competent virus through recombination apply and will be taken into consideration in construction of the set of DNA sequences.

The first DNA sequence used in the first aspect of the invention may be present on separate expression vectors, or it may be present on a single expression vector provided that the vector genome is encoded in a separate transcription unit, The expression vector or vectors will usually be plasmids. Preferably, the DNA constructs encoding the retroviral vector and the required packaging components are administered simultaneously to the patient or delivered simultaneously to the cells being converted to producer cells *in vitro*.

The invention thus comprises the combination of two technologies to produce a method of delivering genes directly to patient/animal tissues such that a long term expression of therapeutic products will be achieved. The essence of the invention is that firstly, using non-viral DNA delivery, combinations of DNA sequences are introduced into the patients cells. The DNA sequence encoding the vector itself, that is the genome of the retrovirus particle, lacks functional *env* and *gag-pol* genes so that the vector is safe and practical. The preferred procedure does not require any removal of cells from the patient. The methods described can however be applied to cells or tissues or organs that are removed from the body and then reimplanted. The combination of DNA sequences is such that when expressed they specify the production of a replication defective retroviral vector genome and the production of the protein components that are required to package that genome to produce retroviral vector particles. The retroviral vector particles are released and then by the process of virus-mediated gene delivery they attach to and enter additional cells and consequently deliver the defective retroviral genome into the cell where it is copied by the particle associated reverse transcriptase and becomes integrated into the genome of those cells. The cell that originally receives the combination of DNA molecules continues to secrete retroviral vector particles for as long as the cell survives or the DNA persists. This creates an extended opportunity for the retroviral vector particles to transduce cells. The retroviral vector particles contain a therapeutic gene which is expressed in the transduced cells. The invention therefore involves establishing a retroviral vector producer cell in the target tissue by the direct delivery of appropriate combinations of DNA sequences.

### Details

DNA is delivered to the cells by any appropriate non-retroviral method including injection, biolistic delivery, carrier mediated delivery. Some of the known methods have already been described above. A preferred delivery method for delivering DNA sequences to cells *in vivo* is liposome-mediated delivery. Whichever method is used for *in vivo* delivery, the DNA sequences will need to be provided in a pharmaceutically acceptable formulation for administering to the patient. Multiple different DNA sequences on separate molecules or a single molecule carrying multiple different sequences are delivered to human/animal cells. These DNA sequences encode the components of a retroviral vector for example the HIT system (Soneoka et al 1995) and the kat system (Finer et al 1994). The DNA sequences encode a retroviral Env protein, a retroviral Gag-Pol protein and a replication defective retroviral genome that is engineered to contain one or more therapeutic genes. Additional sequences may also be included. For example a suicide gene such as HSV Tk might be included on one or all molecules to enable transfected and transduced cells to be destroyed by treatment with drugs such as acyclovir (Plautz et al 1991). The combination of DNA sequences are referred to as the vector production system (VPS). The VPS need not be restricted to the three plasmid systems such as HIT and kat but can comprise any retroviral vector system. Components need not be native retroviral components. For example the *env* gene can be engineered to specify an envelope protein that targets retroviral vector particles to a specific cell type, the vector genome can be engineered to contain gene expression signals that confer special properties on the vector e.g. tissue specific expression, regulated expression and the *gag-pol* gene can be engineered to influence infection and integration for example to deliver DNA into the genome of non-dividing cells or to target DNA to a specific site in the chromosome. The cell that receives the VPS is referred to as an *in situ* retroviral factory (ISRF), it is essentially a retroviral vector producer cell created from one of the patient's own cells. If it is created *in vitro*, using cells removed from the patient, the ISRF will need to be provided in a pharmaceutically acceptable formulation for administering to the patient. The ISRF produces retroviral particles that are released from the cell for as long as the VPS persists, this may be of the order of weeks to months or exceptionally years (Wolff et al 1992). The defective retroviral particles transduce neighbouring cells, referred to as the target cell population (TCP), and deliver the therapeutic gene to those cells as a stably integrated provirus. The TCPs do not produce further virus. The ISRF also expresses the therapeutic gene from the VPS. This combination of non-viral DNA delivery and virus-mediated gene delivery as described allows the dissemination of a therapeutic gene safely, throughout a population of patient's cells.

The invention has a number of advantages which relate to the generation of ISRFs in patients cells both in the body and in tissues removed from the body.

### Advantages

### i) In the patient

1. Increased efficiency of the delivery of retroviral vectors to target cells in the patient because of the local concentration of viral particles.
2. Increased efficiency of the delivery of retroviral vectors because of the extended time period of exposure of cells to viruses. This means that cells at different stages of the cell cycle have the opportunity to cycle into a phase that is optimum for retroviral infection. These cells would not be available as targets in a single dose treatment with retroviral particles.
3. Creation of an ISRF obviates the need to implant a producer cell that has been generated in the laboratory. Such producer cells are different from patients cells and may even be of non-human origin. These cells are rapidly cleared from most sites of implantation in the body and therefore have limited usefulness.
4. The creation of ISRFs dramatically increases the efficiency of non-viral gene therapy methodologies. In these procedures the transient nature of the expression of the therapeutic gene necessitates frequent multiple repeat treatments with DNA. The ISRF will disseminate therapeutic genes to cells that will continue to produce the product for the life-time of the cell. Treatment need therefore be repeated infrequently if at all for some TCPs.
5. Creation of an ISRF obviates the need to surgically remove patient tissues and transduce them with retroviral vectors before reimplantation. This latter procedure does not allow for further dissemination of the therapeutic gene to other cells. It is technically complex and the cells must be subjected to significant manipulation *in vitro* before reimplantation.
6. When applied to the treatment of tumours, the creation of ISRFs increases the probability of obtaining therapeutic gene expression in the majority of tumour cells and hence increases the probability of tumour clearance.
7. The ISRF technology has a variety of therapeutic uses, for example but not restricted to:-
   i) Cystic fibrosis (CF): The VPS is introduced into lung tissue e.g by liposome mediated DNA delivery. The consequently established ISRFs spread the appropriate therapeutic gene e.g. CFTR (cystic fibrosis transmembrane conductance regulator) throughout the pulmonary tissue. This confers extended relief of the pulmonary symptoms of CF.
   ii) Parkinsons disease: The VPS is introduced into cells in the brain by biolistic delivery over a small surgically exposed area. The consequently established ISRFs deliver a retroviral vector to specific cells e.g. glial cells or astrocytes to deliver relevant therapeutic genes e.g. Tyrosine hydroxylase and dopa decarboxylase.
   iii) Alzheimers disease: The VPS is introduced into cells in the brain. The consequently established ISRFs deliver the appropriate therapeutic gene e.g. Nerve growth factor.
   iv) Tumours: The VPS is delivered to the tumour. The consequently established ISRFs deliver a retroviral vector to surrounding tumour cells to deliver relevant therapeutic genes e.g. HSV thymidine kinase (Tk) or foreign histocompatibility antigens.

### ii) In patients tissues ex vivo

Advantages i) 1-4 also apply to *ex vivo* applications of ISRFs
1. Direct transduction of a patient's cells *ex vivo* with retroviral vector particles requires the large scale production of high titre retroviral vectors and is often not very efficient requiring prolonged cell culture and genetic selection of transduced cells. An alternative approach is the cocultivation of patients cells with a producer cell line that has previously been created *in vitro.* This may necessitate the separation of the target cells from the producer cell before reimplantation of patients cells. The present invention describes a method for introducing a therapeutically active gene into a target cell which comprises the step of converting a patient's cell directly into a producer cell. The retroviral vector particles are then transferred from the producer cell, now referred to as an ISRF, to other patient cells and the organ/tissue/cells can be reimplanted with minimal manipulation. The creation of ISRFs therefore obviates the need for cocultivation with non-patient cells or treatment of cells with retroviral vector particles in any *ex vivo* method of gene therapy.

The invention will now be further described in the examples which follow.

### EXAMPLES (for illustrative purposes only)

### Example 1.

### Construction of an ISRF in a Hela cell monolayer

Hela cells are plated into 60cm dishes and allowed to grow to 80% confluence. Plasmid DNA comprising pHIT456, pHIT111 and pHIT60 is prepared for transfer into Hela cells by standard calcium phosphate precipitation and introduced into cells using the extended overlay method as described in detail in Soneoka et al 1995. pHIT456 contains the amphotropic retroviral envelope gene that allows infection of Hela cells, pHIT111 is a defective retroviral genome that contains the *lacZ* gene and pHIT60 contains the MLV *gag-pol* gene. The coexpression of these plasmids results in the production of retroviral vector particles that can transduce target cells with the *lacZ* gene. This is referred to as configuration A. Briefly, 10µg of each plasmid is coprecipitated with calcium phosphate and the resulting precipitate is placed on the Hela cell monolayer. After 24 hrs the medium is removed and replaced with fresh medium. Replicate dishes are taken at 24hr intervals and the cells are fixed and stained with X-gal to detect the expression of β-galactosidase (Sanes et al 1986). In a control experiment 10µg of plasmid pKV469 is used in place of the retroviral vector plasmid pHIT111. pKV469 is a simple eukaryotic cell expression vector that expresses the *lacZ* gene via the CMV-IE promoter. In this three plasmid configuration no retroviral vector particles are produced. This is referred to as configuration B.

Cells that are expressing β-galactosidase are stained blue with X-gal. After 24hrs β-galactosidase is expressed in both configurations from the vector plasmid pHIT111 and from pKV469. When the cells are counted a similar number is observed in each configuration. After 48hrs there is an increase in the number of blue cells in both cases. In configuration B this is due to cell division and adjacent pairs (doublets) of blue cells are observed. In configuration A there is also an increase in the number of cells but this comprises both an increase in doublet cells and an increase in single cells and also the appearance of foci of blue cells. The foci comprise more than two cells which could not result from gene transfer by cell division. The foci appear because virus released from the original cells has infected neighbouring cells. The increase in blue cells in configuration A is more marked after 48 hours with multicellular foci and increased numbers of single cells appearing. This pattern of staining is indicative of one or more rounds of retroviral transduction occurring after the initial transfection of the DNA into the Hela cells. In configuration A, ISRFs are established in the Hela cell monolayer and the *lac*Z gene is disseminated through the target cell population. In configuration B, β-galactosidase expression is resticted to the initially transfected cells and some of their progeny. This experiment establishes that repeated retroviral transduction can occur in a simple homogeneous cell population without the addition of fresh cells as would be the case in a standard cocultivation experiment.

### Example 2

### Dissemination of the lacZ gene throughout the pulmonary tissues of mice

The configuration A and configuration B plasmid sets as above are complexed with cationic liposomes DOTAP or DOTMA/DOPE as described by Alton et al 1993 using 10 to 50µg per plasmid. Liposomes containing DNA are introduced into the lungs of the Edinburgh CF transgenic mouse (Dorin et al 1992) using a jet nebuliser (Alton et al 1993). Mice are sacrificed after 2 days and epithelial cells are harvested by pulmonary lavage. This is repeated for replicate mice at 4 and 14 days. At 14 days lungs are sectioned and sections are stained for the presence of β-galactosidase in pulmonary tissue. In configuaration A the number of blue cells increases to a significantly greater extent than in configuration B and in histological sections foci of blue cells are seen in configuration A but not in configuration B. An ISRF has been established with configuration A and the *lacZ* gene is disseminated through lung tissue.

### Example 3

### Dissemination of the lacZ gene throughout the liver of mice

Mice are subjected to partial hepatectomy. Plasmids in configurations A and B are precipitated by calcium phosphate in the presence of 1µm gold particles. Gold particles are delivered to cells using a biolisitic delivery device. After 2, 4 and 14 days animals are sacrificed and liver sections are stained with X-gal. Foci and scattered blue cells are seen in the liver in configuration A only.

### Example 4

### Dissemination of the lacZ gene throughout the colon of mice

Plasmids in configuration A and B are complexed with cationic liposomes and these are delivered to the colon by instillation. After 2, 4 and 6 days animals are sacrificed and histological sections of the colon are stained with X-gal. Foci and scattered blue cells are seen in colonic epithelium.

### Example 5

### Dissemination of the lacZ gene into the brains of mice.

Plasmids in configuration A and B are introduced in the brains of mice through a surgical window in the cranium. DNA is delivered by a biolistic device. Mice are sacrificed after 4 days and 4 weeks. Foci and scattered blue cells are seen with configuration A only.

### Example 6

### ISRF using human HT1080 cells in culture:

Three-plasmid co-transfections were carried out by calcium-phosphate precipitation as described in Soneoka *et al*. (1995). Plasmid pHIT 60 (MuLV *gag-pol* expression plasmid), pHIT 456 (amphotropic *env* expression plasmid), and pHIT 111 (proviral DNA construct containing the *lacZ* gene) were co-transfected into HT1080 cells in duplicate sets of five 10cm dishes. In the first instance, these cells were maintained for five days. Every day, one dish from one set was fixed and stained with X-gal, and one dish from the other set was harvested and lysed to measure β-galactosidase activity by a colorimetric assay. As a negative control, HT1080 cells were transfected with pHIT 60, pHIT 123 (ecotropic env expression plasmid), and pHIT 111, and the same assays were performed as for the amphotropic producers as described above. Another set of five 10cm dishes was mock-transfected and one dish was harvested everyday to monitor cell growth by counting the number of cells using a hemacytometer. The results obtained are presented in Table 1. The amphotropic virus-producing HT1080 cells showed an increase in *lacZ*-expressing cells and β-galactosidase activity after one day and the levels were maintained thereafter up to day 5. The increase in both *lacZ*-expressing cells and β-galactosidase activity could not be attributed solely to the increase in cell number, since there was no significant cell growth after day 2 and since the ecotropic virus-producing HT1080 cells showed a reduction in the number of *lacZ*-expressing cells (Table 1). In these experiments, polybrene was not used to enhance virus transduction and titers obtained without the use of polybrene at the standard time of 48 hours post-transfection (day 1) were relatively low, approximately 10³ LFU/ml on NIH 3T3 cells for both ecotropic and amphotropic viruses, and also on HT1080 cells with the amphotropic virus. Therefore, it can be concluded that in one 10cm dish containing 5ml of media, up to 5 x 10³ transducible particles were in suspension capable of spreading to surrounding HT1080 cells.

The procedure was repeated but the cells were maintained for 14 days. The transfected cells were passaged on days 5 and 10 at a ratio of 1:5. Again, a similar phenomenon was observed with amphotropic virus-producing HT1080 cells, in that β-galactosidase activity of cells increased after day 1 and was maintained thereafter (Table 2). A significant increase in activity was observed at day 12 (Table 2). This increase was most likely due to proliferation of cells harboring the *lac*Z gene, rather than the spread of retroviral vectors, as no infectious virus was being produced at this stage (Table 2). β-galactosidase activity of ecotropic virus-producing HT1080 cells remained at almost baseline level throughout the 14 days, suggesting that the *lac*Z gene transduced by the amphotropic virus was maintained and stably expressed.

No significant cell growth appeared to occur during the course of either experiment, except only after the first day and probably after passage of the cells on days 5 and 10 in the second experiment (Tables 1 and 2). Cells were transfected at approximately 80% confluency, since some toxicity appears to occur from the calcium-phosphate transfection. Recovery of cells from the transfection may explain the growth of cells between days 1 and 2. By day 2, the cells were nearing confluency, hence, the lack of detectable cell growth. Although the nature of MuLV requires that cells be in an actively-dividing state for infection to occur, the low virus titers produced from day 3 onwards (Table 2) suggests that growth of the cells after day 2 was insignificant.

Taken together these data demonstrate that it is possible to use in situ retroviral factories as effective means of spreading useful retroviral vectors through a population of cells.

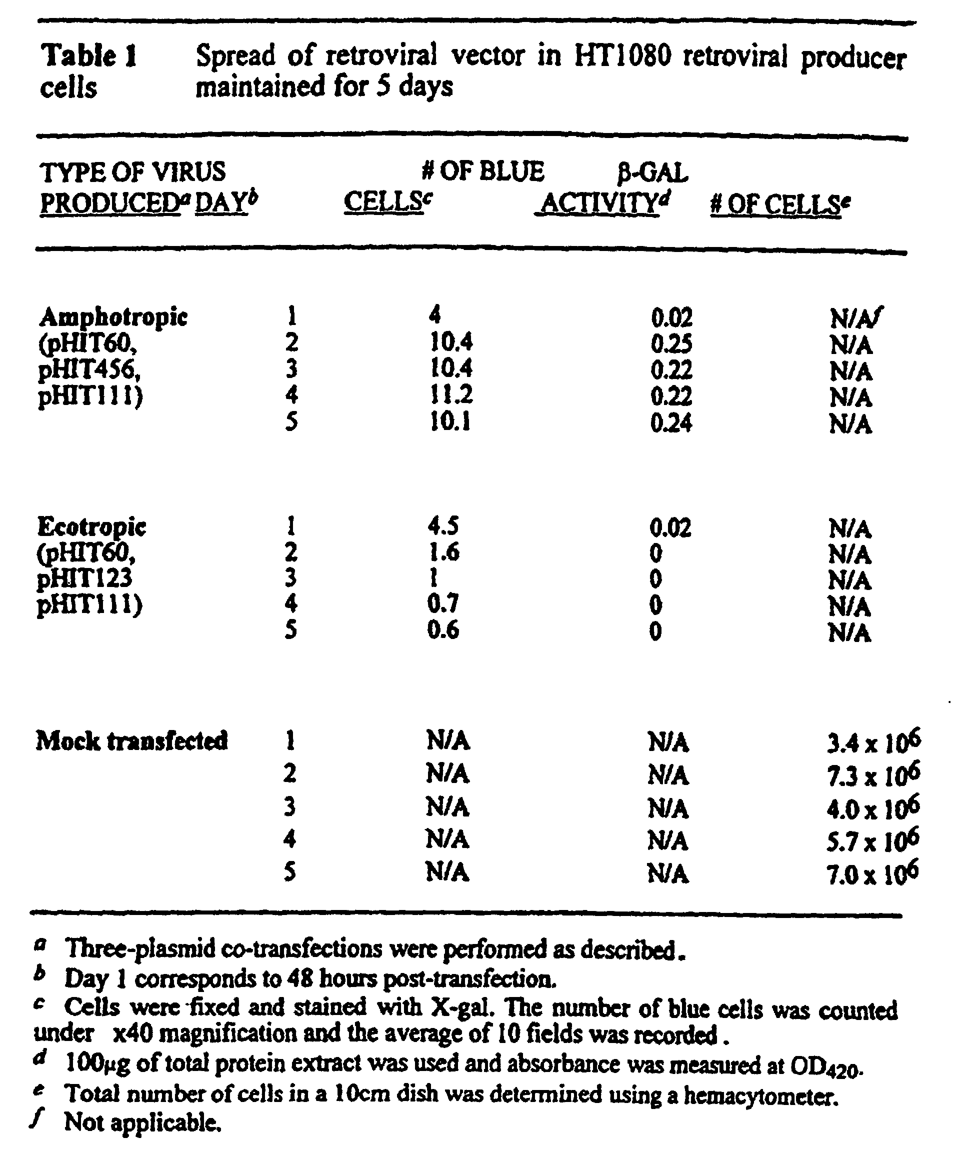

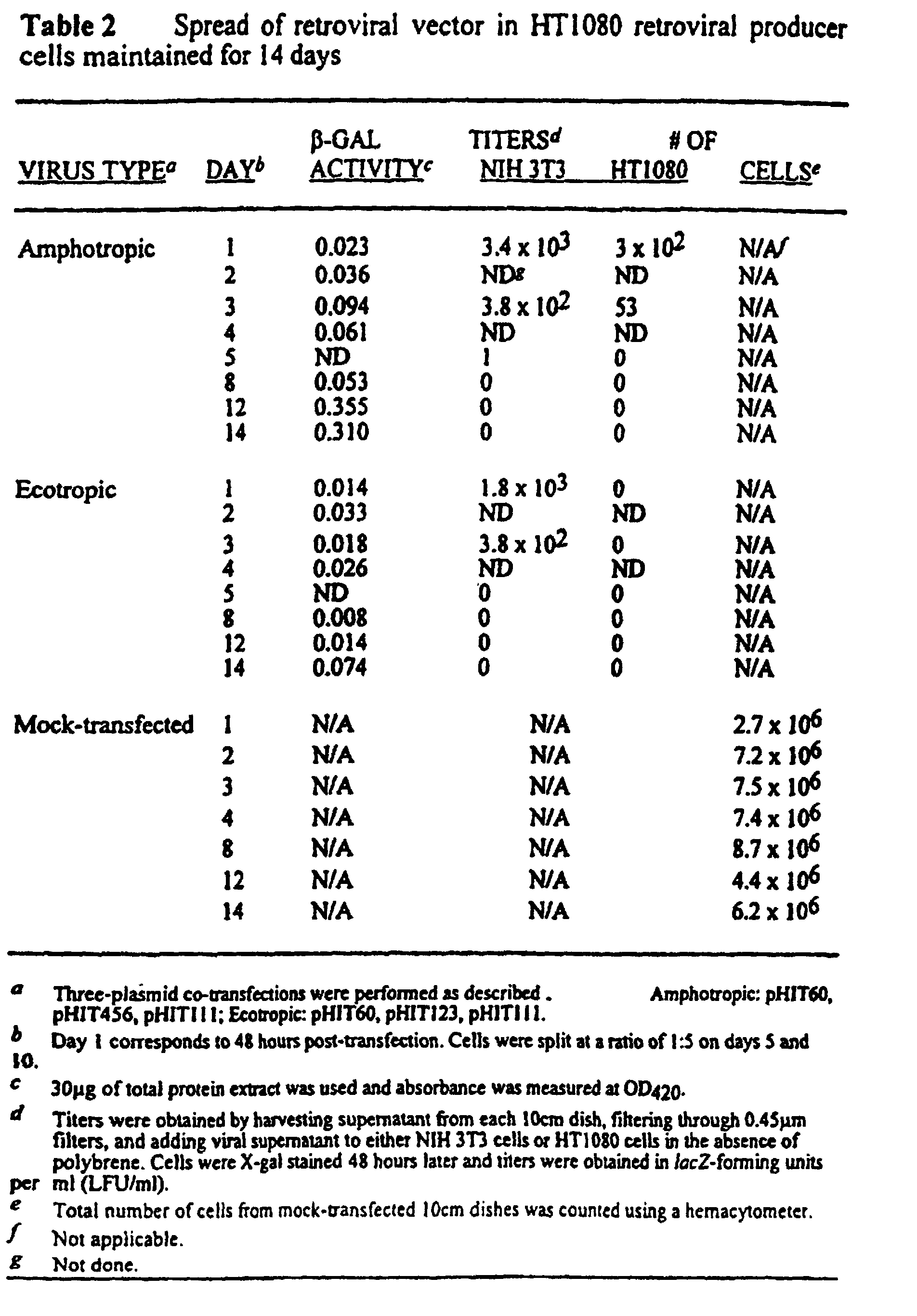

### REFERENCES

Alton, E. et al 1993 Nature Genet. 5, 135
Dorin, J.R. et al 1992. Trans. Res. 1, 101.
Dubensky, T.W. 1984. Proc. Natl. Acad. Sci. 81, 7529
Finer M. et al. 1994. Blood, 83, 43.
Hyde, S.C 1993, Nature 362, 250
Landau, N.R. & Littman, D. 1992. J. Virol. 66, 5110
Ledley, F.D. 1995. Hum. Gene Ther. 6, 1129.
Lever and Goodfellow 1995; Br. Med Buil., 51, 1-242;
Miller, A.D. 1992. Curr. Topics in Microbiol and Immunol. 158, 1-24
Morgan and Anderson. 1993. Annual Review of Biochem., 62, 192
Pear W.S. et al 1993. Proc. Natl. Acad. Sci. 90, 8392
Plautz G. et al. 1991 New Biol.7, 709
Sanes, J.R., et al. 1986 EMBO J., 5 3133.
Short M.P.et al 1990. J. Neuroscience Res. 27, 427
Soneoka Y. et al. 1995. Nucl. Acids Res. 23, 628
Wolff, J.A. 1990. Science, 247, 1465.
Yang, N.S. et al 1990, Proc. Natl. Acad. Sci, 87, 9568.

## Claims

1. The use of a set of DNA sequences comprising:
a first DNA sequence encoding a replication defective retroviral vector, which comprises
(a) a defective retroviral genome lacking functional *env* and functional *gag-pol* genes but having the remaining components essential for retroviral function; and
(b) a therapeutically active gene; and
a second DNA sequence capable of encoding packaging components env and gag-pol, wherein the DNA sequence encoding env is present on a separate construct to the DNA sequence encoding gag-pol
in the manufacture of a medicament for use in a method for introducing the therapeutically active gene into a target cell in a subject which comprises the following steps:
(i) introducing the set of DNA sequences into a cell isolated from the subject which is thereby converted into a producer cell, capable of producing a replication defective retroviral vector;
(ii) reimplanting said producer cell into the subject whereby replication defective retroviral vector particles are produced *in vivo*, and the therapeutically active gene is delivered to the target cell *in vivo*.

2. The use of a set of DNA sequences comprising:
a first DNA sequence encoding a replication defective retroviral vector, which comprises
(a) a defective retroviral genome lacking functional *env* and functional *gag-pol* genes but having the remaining components essential for retroviral function; and
(b) a therapeutically active gene; and
a second DNA sequence capable of encoding packaging components env and gag-pol, wherein the DNA sequence encoding env is present on a separate construct to the DNA sequence encoding gag-pol
in the manufacture of a medicament for use in a method for introducing the therapeutically active gene into a target cell in a subject which comprises the following step:
introducing the set of DNA sequences to a subject's cell by *in vivo* delivery, whereby the subject's cell is converted into a producer cell *in vivo* which is capable of producing replication defective retroviral vector particles, whereby the therapeutically active gene is delivered to the target cell *in vivo*.

3. The use according to claim 1 or 2, in which method the producer cell is of the same type as the target cell.

4. A producer cell capable of producing a replication defective retroviral vector in an infective retroviral particle, the producer cell comprising a set of DNA sequences, comprising:
a first DNA sequence encoding a replication defective retroviral vector, which comprises
(i) a defective retroviral genome lacking functional *env* and functional *gag-pol* genes but having the remaining components essential for retroviral function, and
(ii) at least one therapeutically active gene; and
a second DNA sequence encoding a DNA sequence capable of encoding packaging components env and gag-pol wherein the DNA sequence encoding env is present on a separate construct to the DNA sequence encoding gag-pol
which producer cell is a fresh cell isolated from a subject and, when reimplanted into the subject, is capable of delivering the therapeutically active gene to a target cell within the subject by *in vivo* production of replication defective retroviral vector particles.

5. A producer cell according to claim 4, which is an immune cell.

## Patentansprüche

1. Verwendung eines Satzes von DNA-Sequenzen mit
einer ersten DNA-Sequenz, welche einen bezüglich Replikation defekten retroviralen Vektor codiert, der
(a) ein defektes retrovirales Genom, dem funktionsfähige *env-* und funktionsfähige *gag-pol*-Gene fehlen, der jedoch die übrigen Komponenten hat, die für eine retrovirale Funktion wichtig sind, und
(b) ein therapeutisch aktives Gen
enthält, und
einer zweiten DNA-Sequenz, die in der Lage ist, die Verpackungskomponenten env und gag-pol zu codieren, wobei die DNA-Sequenz, die env codiert, auf einem von der DNA-Sequenz, die gag-pol codiert, getrennten Konstrukt vorhanden ist,
zur Herstellung eines Arzneimittels für die Verwendung in einem Verfahren zum Einführen des therapeutisch aktiven Gens in eine Zielzelle in einem Patienten, welches die folgenden Stufen umfaßt:
(i) Einführen des Satzes von DNA-Sequenzen in eine aus dem Patienten isolierte Zelle, welche dabei in eine Herstellerzelle umgewandelt wird, die in der Lage ist, einen bezüglich Replikation defekten retroviralen Vektor herzustellen,
(ii) Reimplantieren der Herstellerzelle in den Patienten, wobei bezüglich Replikation defekte retrovirale Vektorpartikel *in vivo* hergestellt werden und das therapeutisch aktive Gen *in vivo* an die Zielzelle ausgeliefert wird.

2. Verwendung eines Satzes von DNA-Sequenzen mit
einer ersten DNA-Sequenz, welche einen bezüglich Replikation defekten retroviralen Vektor codiert, der
(a) ein defektes retrovirales Genom, dem funktionsfähige *env-* und funktionsfähige *gag-pol*-Gene fehlen, der jedoch die übrigen Komponenten hat, die für eine retrovirale Funktion wichtig sind, und
(b) ein therapeutisch aktives Gen
enthält, und
einer zweiten DNA-Sequenz, die in der Lage ist, die Verpackungskomponenten env und gag-pol zu codieren, wobei die DNA-Sequenz, die env codiert, auf einem von der DNA-Sequenz, die gag-pol codiert, getrennten Konstrukt vorhanden ist,
zur Herstellung eines Arzneimittels für die Verwendung in einem Verfahren zum Einführen des therapeutisch aktiven Gens in eine Zielzelle in einem Patienten, welches die folgende Stufe umfaßt:
Einführen des Satzes von DNA-Sequenzen in eine Patientenzelle durch *in vivo*-Auslieferung, wobei die Patientenzelle *in vivo* in eine Herstellerzelle umgewandelt wird, welche in der Lage ist, bezüglich Replikation defekte retrovirale Vektorpartikel herzustellen, wobei das therapeutisch aktive Gen *in vivo* an die Zielzelle ausgeliefert wird.

3. Verwendung nach Anspruch 1 oder 2, wobei die Herstellerzelle in dem Verfahren vom gleichen Typ ist wie die Zielzelle.

4. Herstellerzelle, die in der Lage ist, einen bezüglich Replikation defekten retroviralen Vektor in einem infektiösen retroviralen Partikel herzustellen, wobei die Herstellerzelle einen Satz von DNA-Sequenzen enthält mit
einer ersten DNA-Sequenz, die einen bezüglich Replikation defekten retroviralen Vektor codiert, welcher
(i) ein defektes retrovirales Genom, dem funktionsfähige *env-* und funktionsfähige *gag-pol*-Gene fehlen, der jedoch die übrigen Komponenten hat, die für eine retrovirale Funktion wichtig sind, und
(ii) wenigstens ein therapeutisch aktives Gen
enthält, und
einer zweiten DNA-Sequenz, die in der Lage ist, die Verpackungskomponenten env und gag-pol zu codieren, wobei die DNA-Sequenz, die env codiert, auf einem von der DNA-Sequenz, die gag-pol codiert, getrennten Konstrukt vorhanden ist,
wobei die Herstellerzelle eine aus einem Patienten isolierte frische Zelle ist, die, wenn sie in den Patienten reimplantiert wird, in der Lage ist, das therapeutisch aktive Gen an eine Zielzelle in dem Patienten durch *in vivo*-Herstellung von bezüglich Replikation defekten retroviralen Vektorpartikeln auszuliefern.

5. Herstellerzelle nach Anspruch 4, welche eine Immunzelle ist.

## Revendications

1. Utilisation d'une série de séquences d'ADN comprenant :
une première séquence d'ADN codant pour un vecteur rétroviral à réplication défective, qui comprend
(a) un génome rétroviral défectif dépourvu des gènes *env* fonctionnel et *gag-pol* fonctionnel, mais comprenant les constituants restants essentiels pour la fonction rétrovirale ; et
(b) un gène thérapeutiquement actif ; et
une seconde séquence d'ADN capable de coder pour les constituants d'encapsidation *env* et *gag-pol*, la séquence d'ADN codant pour *env* étant présente sur un produit d'assemblage distinct de la séquence d'ADN
codant pour *gag-pol*,
dans la production d'un médicament destiné à être utilisé dans une méthode pour introduire le gène thérapeutiquement actif dans une cellule cible chez un sujet, qui comprend les étapes suivantes :
(i) introduction de la série de séquences d'ADN dans une cellule isolée du sujet, qui est ainsi convertie en une cellule productrice, capable de produire un vecteur rétroviral à réplication détective ;
(ii) réimplantation de ladite cellule productrice dans le sujet, des particules de vecteur rétroviral à réplication défective étant ainsi produites *in vivo*, et le gène thérapeutiquement actif étant délivré à la cellule cible *in vivo*.

2. Utilisation d'une série de séquences d'ADN comprenant :
une première séquence d'ADN codant pour un vecteur rétroviral à réplication défective, qui comprend
(a) un génome rétroviral défectif dépourvu des gènes *env* fonctionnel et *gag-pol* fonctionnel, mais comprenant les constituants restants essentiels pour la fonction rétrovirale ; et
(b) un gène thérapeutiquement actif ; et
une seconde séquence d'ADN capable de coder pour les constituants d'encapsidation *env* et *gag-pol*, ladite séquence d'ADN codant pour *env* étant présente sur un produit d'assemblage distinct de la séquence d'ADN codant pour *gag-pol*,
dans la production d'un médicament destiné à être utilisé dans une méthode pour introduire le gène thérapeutiquement actif dans une cellule cible chez un sujet, qui comprend l'étape suivante :
introduction de la série de séquences d'ADN dans une cellule du sujet par administration *in vivo*, la cellule du sujet étant ainsi convertie en une cellule productrice *in vivo* qui est capable de produire des particules de vecteur rétroviral à réplication défective, le gène thérapeutiquement actif étant ainsi délivré à la cellule cible *in vivo*.

3. Utilisation suivant la revendication 1 ou 2, la cellule productrice dans la méthode mise en oeuvre étant du même type que la cellule cible.

4. Cellule productrice capable de produire un vecteur rétroviral à réplication défective dans une particule rétrovirale infectieuse, la cellule productrice comprenant une série de séquences d'ADN comprenant :
une première séquence d'ADN codant pour un vecteur rétroviral à réplication défective, qui comprend
(i) un génome rétroviral défectif dépourvu des gènes *env* fonctionnel et *gag-pol* fonctionnel, mais comprenant les constituants restants essentiels pour la fonction rétrovirale, et
(ii) au moins un gène thérapeutiquement actif ; et
une seconde séquence d'ADN codant pour une séquence d'ADN capable de coder pour les constituants d'encapsidation *env* et *gag-pol*, la séquence d'ADN codant pour *env* étant présente sur un produit d'assemblage distinct de la séquence d'ADN codant pour *gag-pol*,
cellule productrice qui est une cellule fraîche isolée d'un sujet et qui, lorsqu'elle est réimplantée dans le sujet, est capable de délivrer le gène thérapeutiquement actif à une cellule cible dans le sujet par production *in vivo* de particules de vecteur rétroviral à réplication défective.

5. Cellule productrice suivant la revendication 4, qui est une cellule immunitaire.
